(19) Europäisches Patentamt

European Patent Office

Office européen des brevets

(11) Veröffentlichungsnummer: **0 273 167**
**A1**

(12) # EUROPÄISCHE PATENTANMELDUNG

(21) Anmeldenummer: 87116807.6

(22) Anmeldetag: 13.11.87

(51) Int. Cl.⁴: **A61N 1/04** , A61B 5/04

(30) Priorität: 26.11.86 DE 3640311

(43) Veröffentlichungstag der Anmeldung:
06.07.88 Patentblatt 88/27

(84) Benannte Vertragsstaaten:
AT BE CH DE FR LI NL

(71) Anmelder: **Siemens Aktiengesellschaft Berlin und München**
**Wittelsbacherplatz 2**
**D-8000 München 2(DE)**

(72) Erfinder: **Nützel, Karl-Heinz**
**In der Reuth 102**
**D-8520 Erlangen(DE)**

(54) **Leitfähige Zwischenlage für Elektroden.**

(57) Leitfähige Zwischenlage für Elektroden zum Zuführen oder Abnehmen von elektrischen Signalen am menschlichen oder tierischen Körper, inbesondere für Reizstromelektroden. Sie besteht aus einem Vliesmaterial (3), das zum Applikationsort (1) hin glatt (4), zur Elektrode (2) hin jedoch flauschig (5) ausgebildet ist, wobei die glatte Fläche zwar wasserdurchlässig, gleichzeitig aber schmutzabstoßend ist.

FIG 1

EP 0 273 167 A1

## Leitfähige Zwischenlage für Elektroden

Die Erfindung bezieht sich auf eine leitfähige Zwischenlage für Elektroden gemäß den Merkmalen des Oberbegriffs des Patentanspruchs 1.

Elektroden, insbesondere Reizstromelektroden für die Reizstromtherapie, werden normalerweise nicht direkt, sondern mittels leitfähiger Zwischenlage am menschlichen oder tierischen Körper plaziert. Dadurch paßt sich die Elektrode besser an die Körperoberfläche an. Darüber hinaus ist diese Art Applikation hygienischer, da die Zwischenlage gereinigt oder ausgewechselt werden kann. Die leitfähige Zwischenlage ist z.B. ein mit Leitungswasser oder mit 1 %iger Kochsalzlösung getränktes Papier-, Schwamm-oder Stoffteil, das in Form einer Tasche ausgebildet sein kann (s. z.B. Positionen 62 bis 64 in dem Siemens-Prospekt E 510, Bestell-Nr. M-E510/2056 "Zubehör für Reizstrom-Diagnostik und -Therapie"). Papier und Stoff haben jedoch eine relativ geringe Saugfähigkeit. Ein Schwamm weist zwar eine gute Saugfähigkeit auf, verliert aber einen Teil der Flüssigkeit bereits wieder, insbesondere bei senkrechter Applikation. Schwämme enthalten auch immer einen bestimmten Restanteil an Salzen, die die Leitfähigkeit in unerwünschter Weise erhöhen. Häufig schon nach einmaliger Ingebrauchnahme quillt der Schwamm auch leicht auf, er verformt sich und wird hart und unansehnlich. Auf Papier-, Schwamm-oder Stoffteilen rutscht die Elektrode aber auch leicht am Applikationsort.

Aufgabe vorliegender Erfindung ist es, eine leitfähige Zwischenlage der eingangs genannten Art anzugeben, die gut saugfähig ist, die insbesondere bei senkrechter Applikation, wenn überhaupt, wenig Flüssigkeit verliert und die der Elektrode ein gewisses Maß an Haftung vermittelt.

Die Aufgabe wird erfindungsgemäß mit den kennzeichnenden Merkmalen des Patentanspruchs 1 gelöst.

Das Vliesmaterial gemäß der Erfindung hält Wasser und tropft auch bei senkrechter Anlage nur wenig ab. Auch bei taschenförmiger Ausbildung und Einlagerung der Elektrode im Inneren der Tasche vermittelt das flauschige Vliesmaterial der Elektrode eine solche Haftung, daß sie nicht versehentlich aus der Tasche rutschen kann. Die Praxis zeigt, daß flächige Elektroden selbst dann nicht aus dem Inneren der Tasche herausrutschen können, wenn diese frei senkrecht mit der Öffnung nach unten gehalten wird. Der glatte Teil des Vlieses ist zwar flüssigkeitsdurchlässig, gleichzeitig aber auch schmutzabweisend. Zur Applikationsfläche am Patienten hin ist also die erfindungsgemäße leitfähige Zwischenlage immer feucht und damit gut leitend. Schmutz kann aber nicht in umgekehrter Richtung vom Patienten in die Zwischenlage eindringen.

In einer vorteilhaften Ausgestaltung der Erfindung besteht die Zwischenlage aus medizinischem Vlies, so wie es z.B. bei Wundkissen angewendet wird.

Weitere Vorteile und Einzelheiten der Erfindung ergeben sich aus der nachfolgenden Beschreibung von Ausführungsbeispielen anhand der Zeichnung und in Verbindung mit den Unteransprüchen.

Es zeigen:

Figur 1 ein erstes Ausführungsbeispiel der Erfindung,

Figur 2 ein zweites Ausführungsbeispiel der Erfindung.

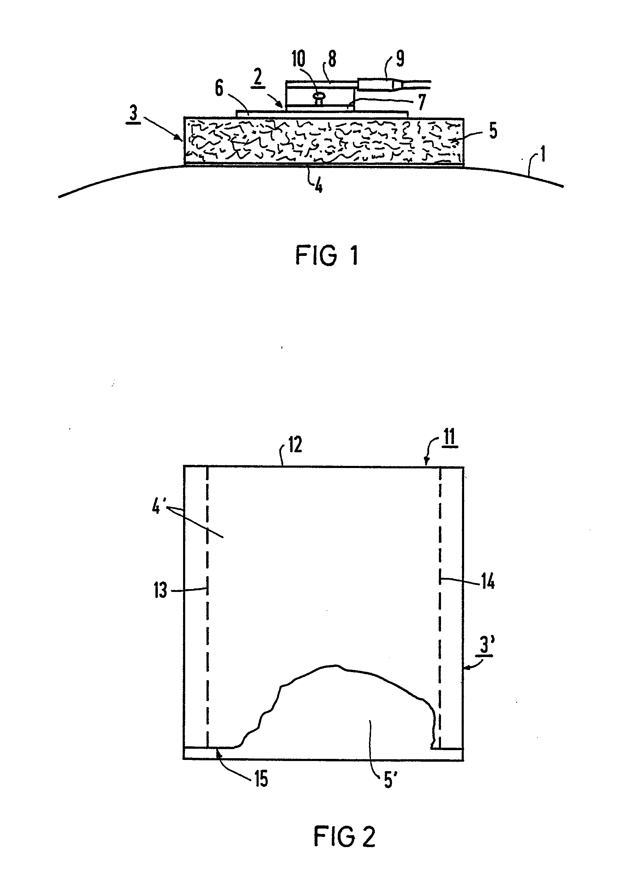

In der Figur 1 liegt zwischen Applikationsort 1 und einer Elektrode 2 ein Stück Vliesmaterial 3, das zum Applikationsort 1 hin glatt (glatte Fläche 4), zur Elektrode 2 hin jedoch flauschig (flauschiges Material 5) ausgebildet ist. Das Vliesmaterial 3 hat medizinische Qualität. Das flauschige Material 5 ist also sehr saugfähig und hält Wasser auch sehr gut bei senkrechter Applikation. Die glatte Fläche 4 ist zum Applikationsort hin für Wasser durchlässig, weist jedoch Schmutz ab, so daß dieser nicht vom Applikationsort in das flauschige Material 5 eindringen kann. Brauchbares medizinisches Vliesmaterial dieser Art ist im Handel erhältlich (z.B. Viledon Typ Nr.T 1555). Es wurde allerdings bisher nur für Wundkissen eingesetzt.

Vor Applikation wird das Vliesmaterial 3 mit Wasser oder mit einer 1 %igen Kochsalzlösung getränkt und, z.B. wie in der Figur 1 dargestellt, appliziert. Die z.B. eine dünne Metallfläche 6 sowie einen Reiter 7 mit Anschlußbuchse 8 für den Stecker eines Reizstromkabels 9 umfassende Elektrode 2 läßt sich mittels eines elastischen Haltebandes (nicht dargestellt), das an einem Knopf 10 des Reiters 7 einknöpfbar ist, am Applikationsort 1 befestigen.

Die Figur 2 zeigt eine Elektrodentasche 11, die wiederum aus Vliesmaterial 3' entsprechend dem Vliesmaterial 3 der Figur 1 gefertigt ist. Dazu wurde das Vliesmaterial 3' an der Kante 12 umgeschlagen und entlang den Strecken 13, 14 genäht. Die Tasche ist dabei außen glatt (glatte Fläche 4') und innen flauschig (flauschiges Material 5'). Durch die Öffnung 15 kann eine Elektrode eingeschoben werden (nicht dargestellt). Nach Anfeuchten der Tasche 11 liegt die Elektrode haftend im Inneren der Tasche. Sie kann selbst bei senkrechter Halterung der Tasche nicht aus dem Inneren herausfallen.

## Ansprüche

1. Leitfähige Zwischenlage für Elektroden zum Zuführen oder Abnehmen von elektrischen Signalen am menschlichen oder tierischen Körper, insbesondere für Reizstromelektroden, **dadurch gekennzeichnet,** daß sie aus einem Vliesmaterial (3, 3') besteht, das zum Applikationsort (1) hin glatt (4, 4'), zur Elektrode (2) hin jedoch flauschig (5, 5') ausgebildet ist, wobei die glatte Fläche zwar wasserdurchlässig, gleichzeitig aber schmutzabstoßend ist.

2. Leitfähige Zwischenlage nach Anspruch 1, **dadurch gekennzeichnet,** daß sie taschenförmig in dem Sinne ausgebildet ist, daß das Äußere der Tasche (11) glatt (4') und das Innere der Tasche flauschig (5') ausgebildet ist.

3. Leitfähige Zwischenlage nach Anspruch 1 oder 2, **dadurch gekennzeichnet,** daß das Vliesmaterial (3, 3') medizinische Qualität aufweist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 029 245 (SIEMENS) <br> * Seite 9, Zeile 5 - Seite 10, Zeile 17; Figuren 6-9 * | 1,3 | A 61 N 1/04 <br> A 61 B 5/04 |
| A | EP-A-0 000 759 (SIEMENS) <br> * Anspruch 16 * | 1,3 | |
| A | CH-A- 434 503 (HAHN) <br> * Spalte 1, Zeilen 11-17 * | 1,2 | |
| A | GB-A- 498 527 (SCHOLL MANUFACTURING CO., LTD) <br> * Seite 2, Zeilen 70-83; Figuren 1,2 * | 1,2 | |
| A | DE-A-3 501 339 (ROBERT BOSCH GmbH) <br> * Anspruch 5 * | 1 | |
| A | DE-A-3 215 960 (DÖLKER-MEDIZINTECHNIK GmbH) <br> * Ansprüche 3,11; Seite 5, Absatz 2; Seite 6, letzter Absatz * | 1,2 | |
| A | DE-A-1 965 195 (ZIMMER) <br> * Anspruch 2 * | 1 | RECHERCHIERTE SACHGEBIETE (Int. Cl.4) <br><br> A 61 N <br> A 61 B |
| A | DE-A-2 727 822 (ZIMMER) <br> * Seite 9, Zeilen 4-5 * | 1 | |
| A | DE-A-2 305 220 (NDM CORP.) <br> * Seite 18, Zeilen 1-5 * | 1 | |
| A | US-A-2 943 627 (HOWELL) <br> * Ansprüche 1,2; Figur 3 * | 1,2 | |
| A | DE-A-2 830 219 (HEITLAND KG) <br> * Seite 4, Absatz 2 * | 1,2 | |

Der vorliegende Recherchenbericht wurde für alle Patentansprüche erstellt

| Recherchenort | Abschlußdatum der Recherche | Prüfer |
|---|---|---|
| DEN HAAG | 10-03-1988 | SCHMIERER U.J. |